(19) Europäisches Patentamt
European Patent Office
Office européen des brevets

(11) Numéro de publication: **0 274 934 B1**

# (12) FASCICULE DE BREVET EUROPEEN

(45) Date de publication du fascicule du brevet: **14.03.90**

(51) Int. Cl.⁴: **C07C 319/08**

(21) Numéro de dépôt: **87402768.3**

(22) Date de dépôt: **04.12.87**

(54) **Procédé de préparation de mercaptoalcools.**

(30) Priorité: **30.12.86 FR 8618323**

(43) Date de publication de la demande: **20.07.88 Bulletin 88/29**

(45) Mention de la délivrance du brevet: **14.03.90 Bulletin 90/11**

(84) Etats contractants désignés: **BE DE ES FR GB IT NL**

(56) Documents cités:
**GB-A- 2 188 632**
**US-A- 4 564 710**

(73) Titulaire: **SOCIETE NATIONALE ELF AQUITAINE (PRODUCTION), Tour Elf 2, Place de la Coupole La Défense 6, F-92400 Courbevoie(FR)**

(72) Inventeur: **Arretz, Emmanuel, 2, rue de Cagnes, F-64000 Pau(FR)**
Inventeur: **Auge, Patrick, Rue Cézanne, F-64140 Lons Billère(FR)**
Inventeur: **Mirassou, Alfred, Cidex No. 15 Poey de Lescar, F-64230 Lescar(FR)**
Inventeur: **Landoussy, Claude, 5, Cours Bosquet, F-64000 Pau(FR)**

(74) Mandataire: **Leboulenger, Jean et al, ATOCHEM Département Propriété Industrielle, F-92091 Paris la Défense 10 Cédex 42(FR)**



Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention a pour objet un procédé perfectionné de préparation de mercaptoalcools, dénommés aussi hydroxymercaptans, hydroxythiols, thioglycols ou encore thioalkylèneglycols,à partir d'époxydes vicinaux et d'hydrogène sulfuré en présence de nouveaux catalyseurs.

La préparation de mercaptoalcools par réaction d'époxydes avec de l'hydrogène sulfuré est bien connue. Ainsi, la production de mercaptoéthanol par l'action d'$H_2S$ sur l'époxyde de l'éthylène, ou oxyde d'éthylène, a déjà fait l'objet d'un certain nombre de travaux. Cependant, lorsqu'on désire préparer des mercaptoalcools à plus de 3 atomes de carbone, de tels procédés ne conviennent pas.

A cet effet, le brevet belge 731 879 propose l'obtention de mercaptoalcools à partir d'époxydes à plus de 3 atomes de carbone, basée sur l'utilisation de catalyseurs fortement basiques, tels que hydroxydes, alcoolates et phénolates alcalins, bases onium tertiaires et/ou quaternaires. Comme autres catalyseurs basiques, le brevet belge 731 881 met en oeuvre diverses amines, et le brevet américain 3 394 192 des trialkyl-amines. Le brevet américain 3 462 496 fait état de catalyseurs du même type : hydroxydes alcalins ou alcalinoterreux, trialkylamines, hydroxydes d'ammonium quaternaire ou encore des sels de chrome d'acides gras.

Plus récemment, la demande de brevet japonais 8059/60 préconise l'addition de sulfure de carbone et d'alcools à la reaction d'un époxyde avec des sulfhydrates de métaux alcalins. En outre, le brevet américain 4 281 202 propose l'emploi de zéolithes sous la forme sodium ou potassium comme catalyseurs, tandis que le brevet français 2 480 281 décrit des catalyseurs constitués de résines échangeuses anioniques, lesquelles ont antérieurement fait l'objet de brevet français 1 359 678.

Cependant, tous ces catalyseurs nécessitent, no tamment dans le cas d'obtention de mercaptoalcools à plus de 3 atomes de carbone, des temps de réaction relativement longs pour n'aboutir qu'à un taux de transformation de l'époxyde et/ou à un rendement médiocre(s) ou insuffisant(s) en produit désiré. En outre, la plupart des procédés de l'art antérieur opèrent en présence de solvants ou autres additifs, même lorsque le milieu réactionnel reste à l'état liquide au course de la réaction.

La présente invention a pour but de remédier aux inconvénients des procédés de l'art antérieur, au moyen de la mise en oeuvre de nouveaux catalyseurs permettant l'obtention de mercaptoalcools avec des rendements et des taux de transformation d'époxydes élevés, tout en autorisant des temps de réaction relativement courts, et en étant dispensée de l'introduction de solvant ou autre additif dans le milieu réactionnel, sauf exceptionnellement dans le cas où ce milieu est très visqueux ou solide.

Conformément à l'invention, on obtient des mercaptoalcools de formule générale :

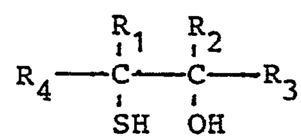

renfermant plus particulièrement jusqu'à 24 atomes de carbone et, de préférence, plus de 3 atomes de carbone, par réaction d'hydrogène sulfuré avec des époxydes vicinaux correspondants, de formule :

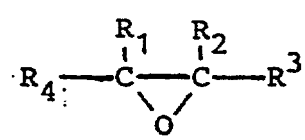

où $R_1$, $R_2$, $R_3$ et $R_4$ peuvent représenter de l'hydrogène, un halogène notamment le chlore, un hydroxy ; un alkyle, haloalkyle, hydroxyalkyle en $C_1$-$C_{22}$ ; alcoxyalkyle , alcoxyhaloalkyle ou alkylthioalkyle en $C_2$-$C_{22}$ ; alcoxyaryles ou alkylthioaryles, notamment alcoxyphényle, éventuellement substitués par un ou des groupements alkyles, halogènes, hydroxyles, alcoxyles, carboxyles ou leurs esters aliphatiques; un groupement contenant un ou plusieurs groupes carboxyliques; alcényle en $C_3$-$C_{22}$ ; cycloalkyle ou cycloalcényle en $C_5C_{12}$ ; arylique ou ou haloarylique en $C_6$-$C_{18}$ ; aralkyle, aryloxyalkyle, arylthioalkyle, alkylaryle, alkylaryloxyalkyle ou alkylarylthioalkyle en $C_7$-$C_{19}$ ; et lorsque $R_1$ et $R_2$ sont pris ensemble ils peuvent représenter un alkylène en $C_3$ à $C_{10}$, caractérisé en ce que la réaction est effectuée en présence d'un catalyseur constitué de guanidine et/ou de dérivés de la guanidine de formule générale :

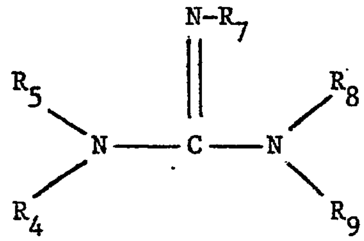

dans laquelle $R_5$, $R_6$, $R_7$, $R_8$ et $R_9$ peuvent représenter de l'hydrogène, un halogène notamment le chlore, un alkyle en $C_1$-$C_8$, alcényle en $C_2$-$C_8$, cycloaliphatique en $C_5$-$C_{12}$, aromatique en $C_6$-$C_{14}$, chacun de ces substituants carbonés peuvent éventuellement posséder un halogène tel que chlore et/ou un groupe fonctionnel tel que hydroxyle et/ou étheroxyde, et/ou de sels de guanidine, en particulier de sels de la guanidine tels que carbonates, chlorhydrates, sulfates, nitrates, thiocyanates, ..., soit sous forme liquide et/ou solide suivant la nature du catalyseur et sa solubilité dans le milieu réactionnel, soit fixé et/ou imprégéné à différents matériaux solides. Ces derniers peuvent être organiques tels que résines polymériques, polymères, copolymères neutres et/ou basiques, ou de nature minérale tels que alumines, silices, alumino-silicates, zéolithes, charbons actifs, oxydes et/ou sels métalliques.

Suivant l'invention, ces guanidines présentent généralement une activité catalytique à partir d'une quantité correspondant à 0,001 % en poids environ par rapport à l'époxyde vicinal, introduit dans le milieu réactionnel. Dans la plupart des cas, la quantité supérieure utile est de l'ordre de 10 % en poids, bien qu'elle puisse être dépassée plus ou moins substantiellement dans certains cas de préparation de mercaptoalcools à partir d'époxydes supérieurs renfermant, par exemple, plus de 18 atomes de carbone. La quantité préférée est, cependant, comprise entre 0,1 et 2% en poids. Dans le cas où une guanidine et/ou un de ses sels est utilisé fixé et/ou imprégné à un matériau solide, les quantités de guanidine introduites sous ces formes dans le milieu réactionnel sont du même ordre, c'est-à-dire de 0,001% à 10% et de préférence de 0,1 0 2% par rapport à l'époxyde vicinal contenu dans le milieu réactionnel.

La transformation d'époxydes en hydroxymercaptans, selon l'invention, est favorisée par le facteur pression. Ainsi, il est avantageux d'opérer entre 4 et 25 bars,ce qui permet d'atteindre des taux de transformation élevés d'époxydes en produits désirés, supérieurs à 90%.

IL est possible de travailler à partir de la température ambiante et,plus particulièrement, entre 20 et 100°C. Mais la température préférée se situe dans l'intervalle de 50 à 80°C.

Selon l'invention, la réaction de l'hydrogène sulfuré avec l'époxyde vicinal est réalisée en mettant en oeuvre un rapport molaire $H_2S$/époxyde supérieur à 1 et, de préférence, compris entre 1,1 et 1,3. Il est à remarquer qu'un excès relativement peu important en $H_2S$, par rapport à l'art antérieur, permet une très bonne sélectivité en hydroxymercaptan, en même temps qu'il assure une conversion très élevée de l'époxyde, souvent supérieure à 90%.

Le temps de réaction dans le procédé de l'invention n'excède généralement pas 120 minutes. Il est le plus souvent compris entre 30 et 60 minutes.

En principe, un solvant n'est pas nécessaire pour effectuer la réaction, contrairement à la plupart des procédés de l'art antérieur. Toutefois, dans le cas d'un mélange réactionnel très visqueux ou solide, par exemple d'époxydes demeurant au moins partiellement à l'état solide dans les conditions opératoires de l'invention, il peut être utile d'adjoindre un solvant inerte convenant habituellement tel que : alcools à bas poids moléculaires comme le méthanol, l'éthanol ou le propanol ; éthers comme le dioxane ; glycols et/ou éthers de glycol. Dans certains cas d'époxydes, des hydrocarbures aliphatiques, ou aromatiques comme le toluène peuvent convenir. Lorsqu'ils s'agit d'époxydes en $C_2$ ou $C_3$, il est possible d'utiliser comme solvant le sous-produit de la réaction avec $H_2S$, c'est-à-dire le thiodiglycol correspondant.

Le mercaptoalcool ainsi obtenu est séparé du milieu réactionnel par les moyens habituels, tels que dégazage préalable de l'$H_2S$ n'ayant pas réagi, suivi de distillation, de préférence sous pression réduite.

La réalisation pratique du procédé de la présente invention peut être effectuée en continu ou en discontinu dans des réacteurs agités ou tubulaires, adaptés au travail sous pression, munis d'équipements permettant un contrôle efficace de la forte exothermicité de la réaction, tels que, entre autres, doubles enveloppes, boucles externes avec échangeurs.

Les exemples suivants ont pour seul but d'illustrer l'invention et il ne doivent donc pas être considérés comme limitatifs.

EXEMPLE 1

On introduit à la temperature ambiante dans un réacteur inoxydable muni d'un agitateur central et d'une double enveloppe, 206,7 g (1,12 mole) d'époxy-1,2 dodécane additionnés de 1,14 g (0,009 mole) de tétraméthylguanidine. Ensuite, on admet progressivement à débit contrôlé de l'hydrogène sulfuré, tandis que la température est portée à 74°C, jusqu'à ce que le rapport molaire $H_2S$/époxy-1,2 dodécane atteigne 1,1. La pression dans le réacteur est maintenue à 10 bars. Une fois l'injection d'$H_2S$ terminée, on maintient le mélange en réaction pendant 10 minutes. La durée totale de la préparation est de 35 minutes.

On récupère sous pression atmosphérique le produit de la réaction, c'est-à-dire le mercapto-1 dodécanol-2 préa lablement débarrassé, par dégazage, de l'$H_2S$ dissous n'ayant pas réagi, suivi d'une distillation à une température de 118-120°C, sous 0,3 mm de Hg. Le rendement en mercapto-1 dodécanol-2 est de 97.2% par rapport à l'époxyde utilisé dont le taux de transformation est de 99%.

A titre de comparaison, on a répété le mode opératoire de l'exemple 1, sauf que la tétraméthylguanidine a été remplacée par de la triéthylamine, représentative d'un catalyseur de l'art antérieur, dans les mêmes proportions molaires par rapport à l'époxy-1,2 dodécane. Malgré une durée de réaction portée à 240 minutes, on n'observe qu'un taux de transformation de l'époxy-1,2 dcdécane de 30%, donc une production en mercapto-1 dodécanol-2 bien inférieure à celle de l'exemple 1.

En reproduisant l'essai précédent mais avec une proportion de triéthylamine quatre fois supérieure, c'est-à-dire de 0,036 mole pour la même quantité d'époxy-1,2 dodécane (206,7 g, soit 1,12 mole), on arrive à améliorer le taux de transformation de l'époxyde (92%), mais il aura fallu un temps de réaction de 180 minutes, c'est-à-dire considérablement plus long que celui de l'exemple 1.

EXEMPLE 2

On réalise le processus opératoire de l'exemple 1, mais avec les modifications suivantes :
Réactif époxyde : époxy-1,2 butane (144 g soit 2 moles)
Catalyseur : diphénylguanidine (3,40 g soit 0,016 mole)
Rapport molaire $H_2S$/époxyde= 1,12
Temps de réaction : 60 minutes
Température : 80°C.
Les résultats sont les suivants :
Taux de transformation de l'époxy-1,2 butane : 96,5%
Rendement en mercapto-1 butanol-2 : 95%.

Dans une autre série d'essais comparatifs exécutés dans les conditions opératoires de l'exemple 1 ci-dessus, mais avec les catalyseurs indiqués ci-après, on observe les résultats résumés dans le tableau suivant.

| Catalyseurs (même quantité que celle de l'exemple 1) | Taux de transformation % de l'époxyde | | Rendement en mercapto-1 dodécanol-2 % |
|---|---|---|---|
| Diéthylamine | 11 | ..... | 8 |
| Triéthylènetétramine | 23 | ..... | 20,5 |
| Hydroxyde de benzyltriméthylammonium | <1 | ..... | |
| Pipéridine | <1 | ..... | |

Toujours à titre comparatif, en appliquant les conditions opératoires de l'exemple 2 décrict plus haut, mais avec les catalyseurs du tableau précédent, on obtient les résultats suivants:

| Catalyseurs (même quantité que celle de l'exemple 2) | Taux de transformation % de l'époxyde | Rendement en mercapto-1 butanol-2 % |
|---|---|---|
| Diéthylamine | 27 | 24 |
| Triéthylènetétramine | 38 | 36 |
| Hydroxyde de benzyltriméthylammonium | 4 | 3,5 |
| Pipéridine | 3,6 | 3 |

On remarque que les 4 catalyseurs connus susmentionnés ne donnent que des résultats significativement inférieurs à ceux procurés au moyen des catalyseurs de la présente invention.

EXEMPLE 3

Une alumine activée, possédant une aire spécifique de 300 $m^2/g$, a été imprégnée de carbonate de guanidine (10 g pour 100 g d'alumine). On a introduit 50 g de ce catalyseur solide dans le réacteur, décrit

dans l'exemple 1, contenant 206,7 g (1,12 mole) d'époxy-1,2 dodécane. On admet progressivement, à débit contrôlé, de l'hydrogène sulfuré, tandis que la température est portée à 74°C, jusqu'à ce que le rapport molaire $H_2S$/époxy-1,2 dodécane atteigne 1,1. La pression dans le réacteur est maintenue à 10 bars. Une fois l'injection terminée, on maintient le mélange en réaction pendant 80 minutes. Le rendement en mercapto-1 dodécanol-2 est de 95% par rapport à l'époxyde utilisé, dont le taux de transformation est de 98%.

EXEMPLE 4

Une série d'expériences a été effectuée, dans laquelle on a utilisé, comme catalyseur de base, une résine anionique échangeuse d'ions, l'Amberlyst A-21. Le même protocole opératoire a été répété avec le réacteur décrit dans l'exemple 1. La charge initiale d'époxy-1,2 dodécane est de 206,7 g (1,12 mole), et l'hydrogène sulfuré est introduit à débit contrôlé, jusqu'à ce que le rapport molaire $H_2$/époxy-1,2 dodécane atteigne 1,1, tandis que la température est portée à 74°C. La pression dans le réacteur est maintenue à 10 bars. Une fois l'injection d'$H_2S$ terminée, on maintient le mélange en réaction pendant le temps nécessaire à la transformation de l'époxy-1,2 dodécane. Le premier essai a consisté à utiliser dans le réacteur 100ml de résine Amberlyst A-21. Après 4 heures de réaction à 74°C, le taux de transformation de l'époxy-1,2 dodécane était de 83 % et le rendement en mercapto-1 dodécanol de 81,4 %.
Pour le deuxième essai on a maintenu la même quantité de résien Amberlyst A-21 (100 ml), mais cette fois l'époxy-1,2 dodécane (206,7 g) a été introduit avec 0,009 mole de triéthylamine. On constate un accroissement très net de la vitesse de la réaction, étant donné qu'au bout de 4 heures, la conversion de l'époxy-1,2 dodécane atteint 94 % et le rendement en mercapto-1, dodécanol-2 est de 92,4 %.
Un essai analogue a été réalisé en remplaçant la triéthylamine par la même quantité molaire de tétraméthylguanidine. L'effet de synergie entre la guanidine et la résine anionique Amberlyst A-21 est encore beacoup plus important, étant donné que l'époxy-1,2 dodécane a été transformé en totalité au bout de 25 minutes.
Enfin, un dernier essai a été effectué avec une charge de résine anionique Amberlyst A-21, qui avait étét au préalable imprégnée de carbonate de guanidine (10%). Dans les mêmes conditions que pour les essais précédents, après 90 minutes de réaction, l'époxy-1,2 dodécane a été converti à 98,5%. Le rendement obtenu en mercapto-1, dodécanol-2 était de 95%.

EXAMPLE 5

Selon le même processus opératoire qu'à l'exemple 1, on fait réagir à 80°C en présence de 0,3 g de tétraméthylguanidine 149,5 g (1,15 mole) de butyl glycidyl éther

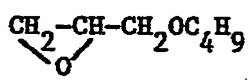

avec de l'hydrogène sulfuré dans un rapport molaire $H_2S$/époxyde égale à 1,13. La durée de réaction est de 45 minutes.

Le taux de transformation de butyl glycidyl éther est de 98 % et on obtient le butoxy-3 mercapto-1 propanol-2 ($HS-CH_2-CHOH-CH_2OC_4H_9$) avec un rendement de 95 %.

Si on remplace la tétraméthylguanidine par 1 g de triéthylamine, le taux de conversion du butyl glycidyl éther n'est que de 89 % et le rendement en mercaptoalcool de 79 % seulement.

EXEMPLE 6

Selon le même processus opératoire qu'à l'exemple 1, on fait réagir à 84°C en présence de 0,6 g de tétraméthylguanidine 214g (1.15 mole) d'éthyl-2 hexyl glycidyl éther

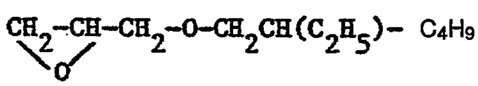

avec de l'hydrogène sulfuré dans un rapport molaire $H_2S$/époxyde égal à 1,15. La réaction dure 40 minutes.

Le taux de transformation de l'époxyde est de 99 % et on obtient le mercaptoalcool $HS-CH_2-CH(OH)-CH_2-OCH_2CH(C_2H_5)-C_4H_9$ avec un rendement de 97 %.

## Revendications

1. Procédé d'obtention de mercaptoalcools de formule générale:

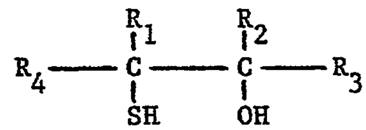

renfermant plus particulièrement jusqu'à 24 atomes de carbone et, de préférence, plus de 3 atomes de carbone, par réaction d'hydrogène sulfuré avec des époxydes vicinaux correspondants, de formule :

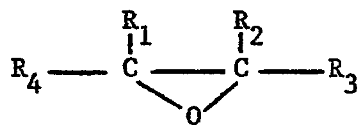

où $R_1$, $R_2$, $R_3$ et $R_4$ peuvent représenter de l'hydrogène, un halogène, un hydroxy ; un alkyle, haloalkyle, hydroxyalkyle en $C_1$-$C_{22}$ ; alcoxyalkyle, alcoxyhaloalkyle ou alkylthioalkyle en $C_2$-$C_{22}$ ; alkoxyaryle ou alkylthioaryle éventuellement substitués par un ou des groupements alkyles, halogènes, hydroxyles, alcoxyles, carboxyles ou leurs esters aliphatiques ; un groupement contenant un ou plusieurs groupes carboxyliques ; alcényle en $C_3$-$C_{22}$ ; cycloalkyle ou cycloalcényle en $C_5$-$C_{12}$ ; arylique ou haloarylique en $C_6$-$C_{18}$ ; aralkyle, aryloxyalkyle, arylthioalkyle, alkylaryle, alkylaryloxyalkyl ou alkylarylthioalkyle en $C_7$-$C_{19}$ ; et lorsque $R_1$ et $R_2$ sont pris ensemble ils peuvent représenter un alkylène en $C_3$ à $C_{10}$, caractérisé en ce que la réaction est effectuée en présence d'un catalyseur constitué de guanidine et/ou de dérivés de la guanidine de formule générale :

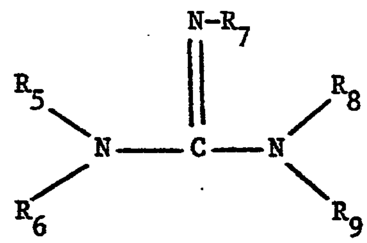

dans laquelle $R_5$, $R_6$, $R_7$, $R_8$ et $R_9$ peuvent représenter de l'hydrogène, un halogène, un alkyl en $C_1$-$C_8$, alcényle en $C_2$-$C_8$, cycloaliphatique en $C_5$-$C_{12}$, aromatique en $C_6$-$C_{14}$, chacun de ces substituants carbonés pouvant éventuellemnt posséder un halogène et/ou groupe fonctionnel, et/ou de sels de telles guanidines, soit sous forme liquide et/ou solide suivant la nature du catalyseur et sa solubilité dans le milieu réactionnel, soit fixé et/ou imprégné à différents matériaux solides.

2. Procédé suivant la revendication 1, caractérisé en ce que la quantité de catalyseur mise en oeuvre est de 0,001 à 10 % en poids par rapport à la quantité d'époxyde introduite dans le milieu réactionnel.

3. Procédé suivant la revendication 1 ou 2, caractérisé en ce que la durée de la réaction n'excède généralement pas 120 minutes, et le plus souvent est comprise entre 30 et 60 minutes.

4. Procédé suivant une des revendications 1 à 3, caractérisé en ce que la réaction est effectuée sous une pression de 4 à 25 bars.

5. Procédé suivant une des revendications 1 à 4, caractérisé en ce que la réaction est effectuée en l'absence de solvant ou autres additifs.

6. Procédé suivant une des revendications 1 à 5, caractérisé en ce que la température de réaction est comprise entre 20 et 100°C et, de préférence, entre 50 et 80°C.

7. Procédé suivant une des revendications 1 à 6, caractérisé en ce que le rapport molaire des réactifs $H_2S$/époxyde est, de préférence, compris entre 1,1 et 1,3.

8. Procédé suivant une des revendications 1 à 4, 6, 7, caractérisé en ce que dans le cas d'un milieu réactionnel très visqueux ou au moins partiellement solide, on met en oeuvre un solvant inerte tel qu'alcools à bas poids moléculaire ; éthers comme le dioxane ; glycols et/ou éthers de glycol ; hydrocarbure aliphatique, en aromatique comme le toluène.

**Patentansprüche**

1. Verfahren zum Erhalten von Mercaptoalkoholen der allgemeinen Formel

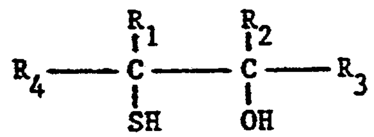

enthaltend insbesondere bis zu 24 Kohlenstoffatome und vorzugsweise mehr als 3 Kohlenstoffatome, durch Umsetzung von Schwefelwasserstoff mit den entsprechenden vicinalen Epoxiden der Formel

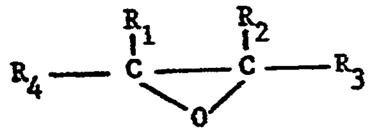

in der $R_1$, $R_2$, $R_3$ und $R_4$ Wasserstoff, Halogen, Hydroxy; ein $C_1$–$C_{22}$-Alkyl, -Halogenalkyl, -Hydroxyalkyl; $C_2$–$C_{22}$-Alkoxyalkyl, -Alkoxyhalogenalkyl oder -Alkylthioalkyl; Alkoxyaryl oder Alkylthioaryl, gegebenenfalls substituiert durch eine oder mehrere Alkyl-, Halogen-, Hydroxyl-, Alkoxyl-, Carboxylgruppe(n) oder deren aliphatische Ester; eine Gruppe, enthaltend eine oder mehrere Carboxylgruppen; $C_3$–$C_{22}$-Alkenyl; $C_5$–$C_{12}$-Cycloalkyl oder -Cycloalkenyl; $C_6$–$C_{18}$-Aryl oder -Halogenaryl; $C_7$–$C_{19}$-Aralkyl, -Aryloxyalkyl, -Arylthioalkyl, -Alkylaryl, -Alkylaryloxyalkyl oder -Alkylarylthioalkyl bedeuten können; und wenn $R_1$ und $R_2$ zusammengenommen werden, sie eine $C_3$–$C_{10}$-Alkylengruppe bilden können, dadurch gekennzeichnet, daß die Reaktion durchgeführt wird in Gegenwart eines Katalysators, bestehend aus Guanidin und/oder Derivaten von Guanidin der allgemeinen Formel

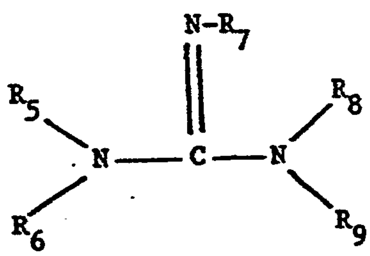

in der $R_5$, $R_6$, $R_7$, $R_8$ und $R_9$ Wasserstoff, ein Halogen, ein $C_1$–$C_8$-Alkyl, $C_2$–$C_8$-Alkenyl, $C_5$–$C_{12}$-cycloaliphatischen Rest, $C_6$–$C_{14}$-Aromaten bedeuten können, wobei jeder dieser kohlenstoffhaltigen Substituenten gegebenenfalls ein Halogen und/oder eine funktionelle Gruppe enthalten kann und/oder den Salzen dieser Guanidine, sei es in flüssiger und/oder fester Form, je nach der Art des Katalysators und seiner Löslichkeit in dem Reaktionsmedium, gegebenenfalls fixiert und/oder imprägniert auf unterschiedlichen festen Materialien.

2. Verfahren nach Anspruch 1, dadurch gekennzeichnet, daß die eingesetzte Menge an Katalysator 0,001 bis 10 Gew.-%, bezogen auf die Menge des eingesetzten Epoxyds in dem Reaktionsmedium, beträgt.

3. Verfahren nach Anspruch 1 oder 2, dadurch gekennzeichnet, daß die Reaktionsdauer im allgemeinen 120 min nicht übersteigt und günstigerweise zwischen 30 und 60 min liegt.

4. Verfahren nach einem der Ansprüche 1 bis 3, dadurch gekennzeichnet, daß die Reaktion unter einem Druck von 4 bis 25 bar durchgeführt wird.

5. Verfahren nach einem der Ansprüche 1 bis 4, dadurch gekennzeichnet, daß die Reaktion in Abwesenheit von Lösungsmittel oder anderen Zusätzen durchgeführt wird.

6. Verfahren nach einem der Ansprüche 1 bis 5, dadurch gekennzeichnet, daß die Reaktionstemperatur zwischen 20° und 100°C, vorzugsweise zwischen 50° und 80°C, liegt.

7. Verfahren nach einem der Ansprüche 1 bis 6, dadurch gekennzeichnet, daß das Molverhältnis der Reaktionspartner $H_2S$:Epoxyd vorzugsweise zwischen 1,1 und 1,3 liegt.

8. Verfahren nach einem der Ansprüche 1 bis 4, 6 und 7, dadurch gekennzeichnet, daß im Falle eines sehr viskosen oder wenigstens teilweise festen Reaktionsmediums man ein inertes Lösungsmittel zusetzt wie Alkohole mit niederem Molekulargewicht, Ether wie Dioxan, Glykole und/oder Glykolether, aliphatische Kohlenwasserstoffe oder aromatische Kohlenwasserstoffe wie Toluol.

## Claims

1. Process for obtaining mercapto alcohols of general formula:

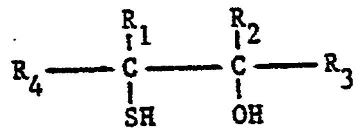

containing, more especially, up to 24 carbon atoms, and preferably more than 3 carbon atoms, by the reaction of hydrogen sulphide with corresponding vicinal epoxides of formula:

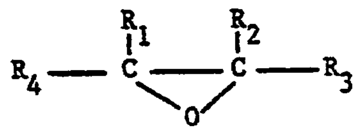

where $R_1$, $R_2$, $R_3$ and $R_4$ can denote hydrogen, a halogen, a hydroxy; a $C_1$–$C_{22}$ alkyl, haloalkyl, hydroxyalkyl; $C_2$–$C_{22}$ alkoxyalkyl, alkoxyhaloalkyl or alkylthioalkyl; alkoxyaryl or alkylthioaryl optionally substituted with one or more alkyl, halogen, hydroxyl or alkoxyl groups or carboxyl groups or aliphatic esters thereof; a group containing one or more carboxyl groups; $C_3$–$C_{22}$ alkenyl; $C_5$–$C_{12}$ cycloalkyl or cycloalkenyl; $C_6$–$C_{18}$ aryl or haloaryl; $C_7$-$C_{19}$ aralkyl, aryloxyalkyl, arylthioalkyl, alkylaryl, alkylaryloxyalkyl or alkylarylthioalkyl; and when $R_1$ and $R_2$ are taken together, they can denote a $C_3$–$C_{10}$ alkylene, characterized in that the reaction is performed in the presence of a catalyst consisting of guanidine and/or of guanidine derivatives of general formula:

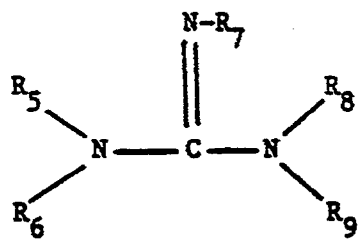

in which $R_5$, $R_6$, $R_7$, $R_8$ and $R_9$ can denote hydrogen, a halogen, a $C_1$–$C_8$ alkyl, $C_2$–$C_8$ alkenyl, $C_5$–$C_{12}$ cycloaliphatic or $C_6$-$C_{14}$ aromatic, it being possible for each of these carbon-containing substituents optionally to possess a halogen and/or a functional group, and/or of salts of such guanidines, either in liquid and/or solid form depending on the nature of the catalyst and its solubility the reaction medium, or attached to and/or impregnated into various solid materials.

2. Process according to claim 1, characterized in that the quantity of catalyst employed is from 0.001 to 10% by weight relative to the quantity of epoxide introduced into the reaction medium.

3. Process according to claim 1 or 2, characterized in that the reaction time does not generally exceed 120 minutes, and is most often between 30 and 60 minutes.

4. Process according to one of claims 1 to 3, characterized in that the reaction is performed under a pressure of 4 to 25 bars.

5. Process according to one of claims 1 to 4, characterized in that the reaction is performed in the absence of a solvent or other additives.

6. Process according to one of claims 1 to 5, characterized that the reaction temperature is between 20° and 100°C and preferably between 50° and 80°C.

7. Process according to one of claims 1 to 6, characterized in that the mole ratio of the reactants $H_2S$/epoxide is preferably between 1.1 and 1.3.

8. Process according to one of claims 1 to 4, 6 and 7, characterized in that, in the case of a very viscous or at least partially solid reaction medium, an inert solvent is employed, such as low molecular weight alcohols; ethers such as dioxane; glycols and/or glycol ethers; an aliphatic hydrocarbon or an aromatic hydrocarbon such as toluene.